# FASCICULE DE BREVET EUROPEEN

(11) **EP 2 776 073 B1**
(45) Date de publication et mention de la délivrance du brevet: **27.03.2019**
(21) Numéro de dépôt: 12777927.0
(22) Date de dépôt: 26.10.2012
(51) Int. Cl.: A61L 2/08

(54) **INSTALLATION DE TRAITEMENT D'ARTICLES PAR BOMBARDEMENT ÉLECTRONIQUE**
ARTIKELBEHANDLUNGSANLAGE MIT ELEKTRONISCHEM BESCHUSS
ARTICLE TREATMENT PLANT USING ELECTRONIC BOMBARDMENT

(30) Priorité: 10.11.2011 FR 1160242
(43) Date de publication de la demande: 17.09.2014
(73) Titulaire: Serac Group, 72400 La Ferté Bernard (FR)
(72) Inventeur: FALLEY, Rémy, F-64990 Urcuit (FR); GUEGUEN, Delphine, F-72400 Cherreau (FR)
(74) Mandataire: Lavialle, Bruno François Stéphane
(86) Numéro de dépôt international: PCT/EP2012/071309
(87) Numéro de publication internationale: WO 2013/068251

(56) Documents cités:
- EP-A1- 2 052 744
- EP-A1- 2 371 397
- EP-A2- 2 138 298
- WO-A2-2009/095182

## Description

La présente invention concerne une installation de traitement d'articles par rayonnement et plus particulièrement par bombardement électronique.

Il est connu de stériliser des récipients en soumettant ceux-ci à un bombardement électronique. Par exemple, EP-A-2 371 397 divulgue une installation de traitement d'articles par rayonnement.

Ce bombardement électronique est réalisé dans une enceinte blindée visant à confiner les rayons X dans une zone dont sont exclus les opérateurs.

A cette fin, une installation de traitement de récipients par bombardement électronique comprend un bâti sur lequel sont montées pour pivoter une étoile d'entrée et une étoile de sortie disposées en regard respectivement d'une entrée et d'une sortie d'une enceinte blindée dans laquelle sont montés une étoile de traitement pivotante et un ou plusieurs émetteurs d'électrons au voisinage de l'étoile de traitement. Les étoiles sont pourvues de moyens de préhension des récipients de sorte que les récipients passent d'une étoile à l'autre de l'entrée vers la sortie.

Pour affaiblir l'énergie des rayons X émis par l'émetteur et les empêcher de sortir de l'enceinte blindée, il est connu de disposer dans l'enceinte des cloisons internes blindées agencées en chicane vers l'entrée et la sortie de l'enceinte blindée. Par un agencement en chicane, on entend que les cloisons internes sont disposées pour interdire un trajet direct des rayons X de l'émetteur jusqu'à l'entrée et jusqu'à la sortie. Ainsi, les rayons X émis par l'émetteur vers les récipients rebondissent contre les parois de l'enceinte blindée et les cloisons internes blindées un nombre suffisant de fois pour avoir perdu l'essentiel de leur énergie avant d'avoir atteint l'entrée et la sortie de l'enceinte blindée.

La mise en place de cloisons internes oblige à éloigner l'étoile de traitement de l'étoile d'entrée et de l'étoile de sortie de sorte qu'il est nécessaire de prévoir deux étoiles intermédiaires montées dans l'enceinte blindée pour tangenter, d'une part, l'étoile de traitement et, d'autre part, l'étoile d'entrée et l'étoile de sortie respectivement au travers de l'entrée et de la sortie.

Si la protection des opérateurs est très bien assurée dans ces installations, elles présentent l'inconvénient d'être très encombrantes en raison de la présence des étoiles intermédiaires dont les dimensions influent directement sur la longueur du chemin à parcourir par les rayons X pour sortir de l'enceinte et donc sur l'étanchéité de l'enceinte.

Un but de l'invention est de fournir un moyen pour protéger les opérateurs intervenant au voisinage d'installation de traitement d'articles par rayonnement tout en limitant l'encombrement de l'installation.

A cet effet, on prévoit, selon l'invention, une installation de traitement d'articles par rayonnement bombardement électronique, comprenant un bâti sur lequel sont montées pour pivoter une étoile d'entrée et une étoile de sortie disposées en regard respectivement d'une entrée et d'une sortie d'une enceinte blindée dans laquelle sont montés au moins une étoile de traitement pivotante et au moins un émetteur d'électrons au voisinage de l'étoile de traitement, les étoiles étant pourvues de moyens de préhension des articles, l'enceinte blindée comportant des cloisons internes blindées agencées en chicanes. L'installation comprend au moins un convoyeur linéaire s'étendant dans l'enceinte blindée respectivement en regard de l'entrée ou de la sortie, le convoyeur linéaire comportant un transporteur s'étendant en anneau allongé autour d'une paroi blindée droite s'étendant selon une direction longitudinale d'une portion droite du transporteur pour former une chicane, la paroi blindée faisant face à ladite entrée ou sortie et ayant une longueur supérieure à une largeur de ladite entrée ou sortie.

Ainsi, la paroi blindée du convoyeur linéaire forme un barrage aux rayonnements sans augmenter l'encombrement longitudinal de l'installation. Pour renforcer le barrage aux rayonnements, il suffit d'allonger la paroi blindée, ce qui peut être fait sans augmenter la distance entre l'entrée et la sortie de l'installation. L'utilisation de convoyeurs linéaires permet en outre une plus grande flexibilité dans l'agencement de l'installation facilitant son implantation dans une ligne de production.

De préférence, l'installation comprend un convoyeur linéaire d'entrée et un convoyeur linéaire de sortie s'étendant dans l'enceinte blindée respectivement en regard de l'entrée et de la sortie, les convoyeurs linéaires comportant un transporteur entourant une paroi blindée s'étendant selon une direction longitudinale du transporteur pour former une chicane à l'entrée et à la sortie respectivement.

D'autres caractéristiques et avantages de l'invention ressortiront à la lecture de la description qui suit de modes de réalisation particuliers non limitatifs de l'invention.

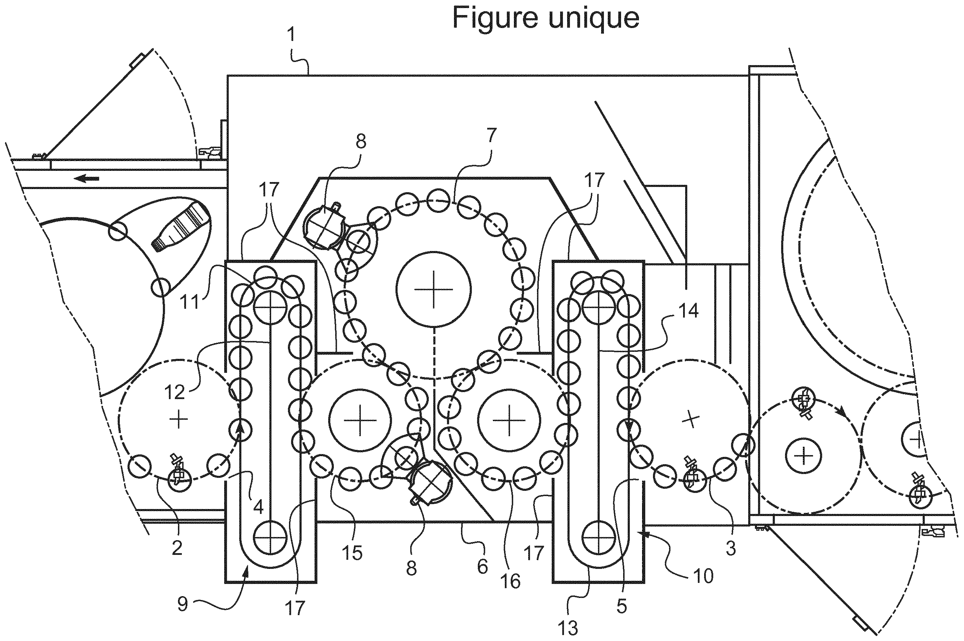

Il sera fait référence à la figure unique annexée représentant schématiquement une installation conforme à l'invention.

En référence à la figure, l'installation de traitement conforme à l'invention est ici agencée pour la stérilisation de récipients par soumission de ceux-ci à un bombardement électronique.

L'installation de l'invention est destinée à prendre place dans une ligne de production, par exemple en aval d'une station de soufflage des récipients et en amont d'une station de remplissage desdits récipients.

L'installation comprend un bâti 1 sur lequel sont montées pour pivoter une étoile d'entrée 2 et une étoile de sortie 3 pourvues chacune de moyens de préhension des récipients tels que des pinces de saisie des récipients par le col. De telles étoiles sont connues en elles-mêmes et ne seront donc pas plus détaillées ici.

Les étoiles d'entrée 2 et de sortie 3 sont disposées en regard respectivement d'une entrée 4 et d'une sortie 5 d'une enceinte blindée 6 dans laquelle est montée pour pivoter une étoile ou plateforme de traitement 7. L'étoile de traitement 7 est pourvue de moyens de préhension des récipients, tels que des pinces de saisie des récipients par le col, et d'émetteurs montés en regard des pinces de saisie. Les pinces et les émetteurs sont montés sur l'étoile pour être mobiles l'un par rapport à l'autre dans une direction verticale pour permettre l'introduction de chaque émetteur dans le récipient supporté par la pince en regard. Une telle étoile de traitement 7 est connue en elle-même et ne sera donc pas plus détaillée ici. Un émetteur fixe 8 est également monté dans l'enceinte blindée 6 au voisinage de la périphérie de l'étoile de traitement 7 pour émettre un faisceau d'électrons vers les récipients qui défilent en regard de l'émetteur fixe 8 lors de la rotation de l'étoile de traitement 7.

Un convoyeur linéaire d'entrée 9 et un convoyeur linéaire de sortie 10 s'étendent dans l'enceinte blindée 6 respectivement en regard de l'entrée 4 et de la sortie 5 de manière à tangenter respectivement l'étoile d'entrée 2 et l'étoile de sortie 3.

Le convoyeur linéaire d'entrée 9 comporte un transporteur 11 s'étendant en anneau allongé autour d'une paroi blindée 12 faisant sensiblement face à l'entrée 4.

Le convoyeur linéaire de sortie 10 comporte un transporteur 13 s'étendant en anneau allongé autour d'une paroi blindée 14 faisant sensiblement face à la sortie 5.

Les transporteurs 11, 13 sont pourvus de moyens de support des récipients qui sont connus en eux-mêmes et permettent de transférer les récipients de l'étoile d'entrée 2 au convoyeur linéaire d'entrée 9 et du convoyeur linéaire de sortie 10 à l'étoile de sortie 3.

La paroi blindée 12, 14 a une longueur (mesurée parallèlement à la grande dimension du convoyeur linéaire considéré) supérieure à la largeur (mesurée parallèlement à la grande dimension du convoyeur linéaire considéré) de l'entrée 4 et de la sortie 5 respectivement. Les parois blindées 12, 14 forment ainsi une chicane respectivement à l'entrée 4 et à la sortie 5 de l'enceinte blindée 6.

Des étoiles intermédiaires 15, 16 sont montées dans l'enceinte blindée 6 pour pivoter. L'étoile intermédiaire 15 est montée entre l'étoile de traitement 7 et le convoyeur linéaire d'entrée 9 pour tangenter ceux-ci. Les étoiles intermédiaires 15, 16, connues en elles-mêmes, sont pourvues de moyens de préhension des récipients, tels que des pinces de saisie des récipients par le col. Un émetteur fixe 8 est aussi monté au voisinage de la périphérie de l'étoile intermédiaire 15.

Les points de tangence de chaque convoyeur linéaire 9, 10 avec les étoiles qui l'entourent se trouvent sur les grands côtés du transporteur 11, 13 de telle manière les longueurs de transporteur s'étendant de part et d'autre des points de tangence soient identiques l'une à l'autre. En variante, ces longueurs peuvent être différentes.

L'enceinte blindée 6 comporte des cloisons internes 17 blindées agencées en chicanes. Les cloisons internes 17 s'étendent depuis les parois de l'enceinte blindée 6 de manière que les étoiles et les convoyeurs linéaires soient séparés les uns des autres par des cloisons internes 17 sauf dans la zone de tangence où les cloisons internes laissent des ouvertures de passage dont les dimensions sont juste suffisantes pour permettre respectivement le passage des récipients du convoyeur linéaire d'entrée 9 à l'étoile intermédiaire 15, de l'étoile intermédiaire 15 à l'étoile de traitement 7, de l'étoile de traitement 7 à l'étoile intermédiaire 16, de l'étoile intermédiaire 16 au convoyeur linéaire de sortie 10.

Par blindée, on entend que la paroi ou cloison est étanche aux électrons et rayons X. La paroi ou cloison blindée comprend ainsi une couche de plomb recouverte d'une feuille d'acier inoxydable.

Bien entendu, l'invention n'est pas limitée aux modes de réalisation décrits mais englobe toute variante entrant dans le champ de l'invention telle que définie par les revendications.

En particulier, l'installation peut ne pas comprendre d'étoile intermédiaire, les convoyeurs linéaires venant tangenter l'étoile de traitement.

L'installation peut comprendre un nombre différent d'étoiles intermédiaires ou d'étoiles de traitement.

L'installation peut comprendre des émetteurs embarqués sur l'étoile de traitement et plusieurs émetteurs fixes, uniquement un ou plusieurs émetteurs fixes, ou uniquement des émetteurs embarqués sur l'étoile de traitement.

Les étoiles peuvent avoir une structure différente de celle décrite notamment en ce qui concerne la préhension des récipients.

L'invention peut être appliquée à d'autres articles que des récipients et/ou à d'autres types de rayonnement.

## Revendications

1. Installation de traitement d'articles par rayonnement, comprenant un bâti (1) sur lequel sont montées pour pivoter une étoile d'entrée (2) et une étoile de sortie (3) disposées en regard respectivement d'une entrée (4) et d'une sortie (5) d'une enceinte blindée (6) dans laquelle sont montés au moins une étoile de traitement (7) pivotante et au moins un émetteur d'électrons (8) au voisinage de l'étoile de traitement, les étoiles étant pourvues de moyens de préhension des articles, l'enceinte blindée comportant des cloisons internes blindées (17) agencées en chicanes, **caractérisée en ce qu'**elle comprend au moins un convoyeur linéaire d'entrée (9, 10) s'étendant dans l'enceinte blindée en regard de l'entrée ou de la sortie, le convoyeur linéaire comportant un transporteur (11, 13) s'étendant en anneau allongé autour d'une paroi blindée (12, 14) droite s'étendant selon une direction longitudinale d'une portion droite du transporteur pour former une chicane, la paroi blindée faisant face à ladite entrée ou sortie et ayant une longueur supérieure à une largeur de ladite entrée ou sortie.

2. Installation selon la revendication 1, comprenant un convoyeur linéaire d'entrée (9) et un convoyeur linéaire de sortie (10) s'étendant dans l'enceinte blindée respectivement en regard de l'entrée et de la sortie, les convoyeurs linéaires comportant un transporteur (11, 13) entourant une paroi blindée (12, 14) s'étendant selon une direction longitudinale du transporteur pour former une chicane à l'entrée et à la sortie respectivement.

3. Installation selon la revendication 1, dans laquelle au moins une étoile intermédiaire (15) est montée dans l'enceinte blindée (6) entre l'étoile de traitement (7) et le convoyeur linéaire (9, 10).

4. Installation selon la revendication 1, dans laquelle les cloisons internes blindées (17) séparent l'étoile de traitement du convoyeur linéaire en laissant une ouverture de passage des articles.

## Patentansprüche

1. Anlage zur Behandlung von Artikeln mittels Strahlung, umfassend ein Gestell (1), an dem ein Einlassstern (2) und ein Auslassstern (3), die gegenüber einem Einlass (4) bzw. einem Auslass (5) einer abgeschirmten Einfassung (6) angeordnet sind, drehbar gelagert sind, in der mindestens ein sich drehender Behandlungsstern (7) und mindestens ein Elektronenemitter (8) nahe dem Behandlungsstern montiert sind, wobei die Sterne mit Greifmitteln zum Greifen der Artikel versehen sind, wobei die abgeschirmte Einfassung abgeschirmte Innenwände (17) umfasst, die als Ablenkplatten ausgebildet sind, **dadurch gekennzeichnet, dass** sie mindestens einen linearen Einlassförderer (9, 10) umfasst, der sich in der abgeschirmten Einfassung gegenüber dem Einlass oder dem Auslass erstreckt, wobei der lineare Förderer eine Transporteinrichtung (11, 13) umfasst, die sich als länglicher Ring um eine abgeschirmte geradlinige Wand (12, 14) erstreckt, die sich in einer Längsrichtung eines geradlinigen Abschnittes der Transporteinrichtung erstreckt, um eine Ablenkplatte zu bilden, wobei die abgeschirmte Wand dem genannten Einlass oder Auslass gegenüberliegt und eine Länge hat, die größer als eine Breite des genannten Einlasses oder Auslasses ist.

2. Anlage nach Anspruch 1, umfassend einen linearen Einlassförderer (9) und einen linearen Auslassförderer (10), die sich in der abgeschirmten Einfassung gegenüber dem Einlass bzw. dem Auslass erstrecken, wobei die linearen Förderer eine Transporteinrichtung (11, 13) umfassen, die eine abgeschirmte Wand (12, 14) umgibt, die sich in einer Längsrichtung der Transporteinrichtung erstreckt, um eine Ablenkplatte am Einlass bzw. am Auslass zu bilden.

3. Anlage nach Anspruch 1, bei der mindestens ein Zwischenstern (15) in der abgeschirmten Einfassung (6) zwischen dem Behandlungsstern (7) und dem linearen Förderer (9, 10) montiert ist.

4. Anlage nach Anspruch 1, bei der die abgeschirmten Innenwände (17) den Behandlungsstern von dem linearen Förderer trennen und dabei eine Öffnung zum Durchtritt der Artikel lassen.

## Claims

1. Installation for treating articles with radiation, the installation comprising a structure (1) having pivotally mounted thereon an inlet starwheel (2) and an outlet starwheel (3) respectively arranged facing an inlet (4) and an outlet (5) of a shielded enclosure (6) in which there are mounted at least one pivotal treatment starwheel (7) and at least one electron emitter (8) in the vicinity of the treatment starwheel, the starwheels being provided with article-gripper means, the shielded enclosure having internal shielded partitions (17) arranged as baffles, the installation being **characterized in that** it includes at least one linear inlet conveyor (9, 10) extending inside the shielded enclosure facing the inlet or the outlet, the linear conveyor having a transporter (11, 13) extending as an elongate ring around a straight shielded wall (12, 14) extending in a longitudinal direction of a straight portion of the transporter in order to form a baffle, the shielded wall facing said inlet or outlet and having a length superior to a width of said inlet or oulet.

2. Installation according to claim 1, including a linear inlet conveyor (9) and a linear outlet conveyor (10) extending inside the shielded enclosure respectively facing the inlet and the outlet, the linear conveyors each comprising a transporter (11, 13) surrounding a shielded wall (12, 14) extending in a longitudinal direction of the transporter in order to form a baffle at the inlet and a baffle at the outlet, respectively.

3. Installation according to claim 1, wherein at least one intermediate starwheel (15) is mounted inside the shielded enclosure (6) between the treatment starwheel (7) and the linear conveyor (9, 10).

4. Installation according to claim 1, wherein the shielded internal partitions (17) separate the treatment starwheel from the linear conveyor while leaving an opening for passing the articles.
